# DEMANDE DE BREVET EUROPEEN

(11) **EP 0 819 697 A2**
(43) Date de publication de la demande: **21.01.1998**
(21) Numéro de dépôt: 97401198.3
(22) Date de dépôt: 30.05.1997
(51) Int. Cl.: C07H 7/02, A62D 1/00

(54) **1-C-perfluoroalkyl glycosides, procédé de préparation et utilisations**

(30) Priorité: 20.06.1996 FR 9607692
(71) Demandeur: CECA S.A., 92800 Puteaux (FR)
(72) Inventeur: Lavaire, Sandrine, 51100 Reims (FR); Platier-Royon, Richard, 51100 Reims (FR); Portella, Charles, 51350 Cormontreuil (FR)
(74) Mandataire: Haicour, Philippe

(57) **Abrégé**

L'invention concerne des 1-*C*-perfluoroalkyl glycosides constitués d'un monosaccharide dont le carbone anomérique porte un radical perfluoroalkyle et un groupe hydroxyle.

Elle concerne également un procédé de préparation desdits glycosides qui consiste à :
a - faire réagir une aldonolactone avec un agent de protection des groupes hydroxyles,
b - faire réagir le produit de l'étape a) avec un composé de formule R_{F}-M dans laquelle R_{F} représente un radical perfluoroalkyle, linéaire ou ramifié, renfermant de 2 à 12 atomes de carbone, et M représente Li ou MgX, X étant un halogène,
c - et déprotéger lesdits groupes hydroxyles.

Les 1-*C*-perfluoroalkyl glycosides peuvent être utilisés comme agents tensioactifs et comme agents d'extinction des feux.

## Description

La présente invention concerne des 1-C-perfluoroalkyl glycosides, leur procédé de préparation et leur utilisation dans le domaine des tensioactifs.

Les sucres perfluoroalkylés sont connus pour leur propriétés tensioactives et leur capacité à transporter l'oxygène. Cette dernière propriété est mise à profit dans le domaine biomédical, notamment pour la préparation de substituts sanguins injectables.

Les sucres perfluoroalkylés ont fait l'objet de divers travaux dont on peut citer :
- l'article de RIESS J.G. et GREINER J. (Carbohydrates as Organic Raw Materials II, pp. 209-259, Ed. VCH (1993)) qui décrit des sucres perfluoroalkylés comprenant une tête hydrophile de nature glucidique, un élément de jonction (par exemple ester, éther, amide ou phosphoester), un espaceur hydrocarboné et une queue perfluoroalkylée,
- l'article de EL GHOUL M. et al. (J. Fluorine Chem., vol. 59, pp. 107-112 (1992)) qui propose notamment des N-[2-(F-alkyl)éthyl]-lactosylamides,
- et la demande de brevet EP-A-375610 qui décrit des perfluoroalkylthioglycosides de formule R_{F}-E-S-saccharide dans laquelle R_{F} représente un radical en C₁-C₁₈, E est un groupe connecteur et le saccharide est un oligosaccharide renfermant 1 à 30 unités d'un sucre en C₅-C₇.

Dans les documents de l'art antérieur qui viennent d'être cités, on constate que le carbone anomérique porte d'une part un substituant composé d'un bras espaceur et d'un radical perfluoroalkyle, et d'autre part un atome d'hydrogène.

La présente invention concerne de nouveaux perfluoroalkyl-C- glycosides, lesquels glycosides sont caractérisés en ce qu'ils sont constitués d'un monosaccharide dont le carbone anomérique est lié directement à un radical perfluoroalkyle et à un groupe hydroxyle.

Un autre objet de l'invention concerne un procédé de préparation desdits glycosides comprenant les étapes suivantes :
- protection des groupes hydroxyles d'une aldonolactone,
- réaction avec un réactif perfluoroalkylé,
- et déprotection desdits groupes hydroxyles.

Un autre objet de l'invention concerne l'utilisation des perfluoroalkyl-*C*-glycosides en tant qu'agents tensioactifs et agents d'extinction des feux.

Les perfluoroalkyl-C-glycosides selon l'invention sont constitués d'un monosaccharide dont le carbone 1 porte un radical perfluoroalkyle et un groupe hydroxyle.

Le radical perfluoroalkyle est généralement choisi parmi les radicaux linéaires ou ramifiés renfermant de 2 à 12 atomes de carbone, et de préférence 2 à 8 atomes de carbone.

Le monosaccharide est généralement un ose renfermant 4 à 7 atomes de carbone. De préférence, l'ose est l'érythrose, le glucose, le galactose, le gulose ou le mannose, et avantageusement le glucose.

Les 1-C-perfluoroalkyl glycosides selon l'invention peuvent être préparés par un procédé qui consiste à :
**a** - faire réagir une aldonolactone avec un agent de protection des groupes hydroxyles,
**b** - faire réagir le produit de l'étape a) avec un composé de formule R_{F}-M dans laquelle R_{F} représente un radical perfluoroalkyle, linéaire ou ramifié renfermant de 2 à 12 atomes de carbone, et M représente Li ou MgX, X étant un halogène, de préférence Br,
**c** - et déprotéger lesdits groupes hydroxyles.

Dans la mise en oeuvre de l'étape a), on utilise en général une aldonolactone renfermant de 4 à 7 atomes de carbone, et de préférence 6 atomes de carbone. A titre d'exemples, on peut citer l'érythrono-1,4-lactone, la glucono-1,5-lactone, la glucoheptono-1,4-lactone, la galactono-1,4-lactone, la gulono-1,4-lactone et la mannono-1,4-lactone. De préférence, on utilise la glucono-1,5-lactone, et avantageusement la D-glucono-1,5-lactone.

L'agent de protection est généralement choisi parmi les composés susceptibles de former des groupements éthers et/ou cétaliques avec les groupes hydroxyles libres de l'aldonolactone.

Pour obtenir les dérivés éthers, on peut utiliser un halogénure d'alkyle, par exemple le bromure ou le chorure de benzyle.

Pour obtenir les dérivés préférés persilylés, on peut utiliser un halogénure de trialkylsilyle, par exemple le chlorotriméthylsilane et/ou un hexaalkyldisilazane, par exemple l'hexaméthyldisilazane. De préférence, on utilise un mélange de chlorotriméthylsilane et d'hexaméthyldisilazane.

La réaction est généralement réalisée en présence d'une base, par exemple la pyridine ou la triéthylamine. De préférence, on utilise la pyridine.

La réaction est généralement mise en oeuvre à une température de l'ordre de 20°C et pendant une durée pouvant varier de 1 à 6 heures.

Pour obtenir les dérivés cétaliques, on peut utiliser un composé carbonylé, par exemple l'acétone, ou un éther d'énol, par exemple le 2-méthoxypropène. De préférence, on utilise le 2-méthoxypropène.

La réaction est généralement réalisée en présence d'un solvant, par exemple l'acétone ou le diméthylformamide, et d'un catalyseur acide, par exemple l'acide para-toluènesulfonique, à une température comprise de l'ordre de 0°C et pendant une durée pouvant varier de 20 à 48 heures.

En général, l'agent de protection et la base sont utilisés en excès par rapport à la lactone.

Les dérivés mixtes éther/cétal peuvent être obtenus en mettant en oeuvre successivement les étapes de préparation du cétal et de l'éther décrites ci-avant. De préférence, le dérivé comporte des liaisons éthers en position 2 et 3 et cétaliques en position 4 et 6 (cas de la glucono-1,5-lactone).

Le produit obtenu à l'issue de l'étape a) peut être constitué d'un ou plusieurs des dérivés précités. De préférence, le produit est un dérivé persilylé, un dérivé mixte (2,3-éthersilylé, 4,6-cétal) ou un mélange de ces dérivés (cas de la glucono-1,5-lactone).

Le composé R_{F}-M mis en oeuvre à l'étape b) est généralement obtenu selon les méthodes connues de l'homme du métier, par exemple à partir de R-M et R_{F}-Y, R représentant un radical alkyle ou aryle ayant 1 à 6 atomes de carbone, R_{F} ayant la signification donnée ci-avant et Y étant I ou Br (voir l'article de BURTON D.J. et al., Tetrahedron, vol. 48, N°2, pp. 189-275, 1992).

En général, le rapport du nombre d'équivalent en mole de R_{F}-M au nombre d'équivalent en mole de la lactone varie de 1 à 1,5. De préférence, le rapport est égal à 1,1.

La réaction est généralement réalisée à une température comprise entre -50°C et -10°C, de préférence de l'ordre de -45°C, et pendant une durée pouvant varier de 1 à 6 heures.

La déprotection mise en oeuvre à l'étape c) est généralement réalisée par hydrogénolyse (dérivés peralkylés, en particulier perbenzylés) dans les conditions connues de l'homme du métier ou par hydrolyse (dérivés éthers, cétaliques et mixtes).

Dans le cas des dérivés éthers tels que les dérivés persilylés, l'hydrolyse totale peut être réalisée selon deux alternatives.

La première alternative consiste à faire réagir le produit obtenu à l'étape b) avec un fluorure tel qu'un fluorure de tétraalkylammonium ou un fluorure alcalin, par exemple de césium ou de potassium, en présence d'un solvant tel qu'un alcool. De préférence, on utilise le fluorure de tétrabutylammonium.

La deuxième alternative consiste à dissoudre le produit obtenu à l'étape b) dans un alcool, par exemple le méthanol ou l'éthanol, et chauffer au reflux. De préférence, on utilise le méthanol.

Dans le cas des dérivés cétaliques, l'hydrolyse totale est généralement réalisée au moyen d'une résine acide, par exemple Amberlyst 15 wet H⁺ commercialisée par ALDRICH, avantageusement en présence d'un mélange eau/solvant miscible à l'eau, de préférence eau/éthanol, chauffé au reflux.

Dans le cas des dérivés mixtes, l'hydrolyse peut être sélective ou totale.

Pour réaliser l'hydrolyse sélective, on met généralement en contact le dérivé mixte avec un fluorure dans les conditions décrites ci-avant pour les dérivés persilylés. Le dérivé cétalique 1-C-perfluoroalkylé ainsi obtenu peut éventuellement être purifié, par exemple par recristallisation. L'hydrolyse finale peut être réalisée au moyen d'une résine acide dans les conditions décrites ci-avant pour les dérivés cétaliques.

Pour réaliser l'hydrolyse totale, on soumet généralement le dérivé mixte obtenu à l'étape b) à l'action d'une résine acide dans les conditions décrites ci-avant pour les dérivés cétaliques. Le 1-*C*-perfluoroalkyl glycoside ainsi obtenu peut éventuellement subir une étape de purification, par exemple par chromatographie sur gel de silice.

Les 1-C-perfluoroalkyl glycosides selon l'invention sont susceptibles de nombreuses applications, par exemple en tant qu'agents tensioactifs, notamment dans le domaine des cosmétiques, et agents d'extinction des feux.

Les exemples qui suivent permettent d'illustrer l'invention.

Dans les exemples suivants, on utilise les méthodes d'analyse ci-après :
- Résonance magnétique nucléaire (RMN)
   Les déplacements chimiques sont exprimés en ppm et les constantes de couplage J en Hz.
   Les composés sont solubilisés dans CDCl₃ (composés 1 à 3), CD₃COCD₃ (composés 4) et CD₃OD (composés 5).
   - RMN ¹H : appareil BRÜCKER ; 250 MHz (composés 1 à 4) et 500 MHz (composés 5)
   - RMN ¹³C : appareil BRÜCKER ; 62,89 MHz (composés 1 à 4) et 125 MHz (composés 5)
   - RMN ¹⁹F : appareil BRÜCKER ; 235,36 MHz
- Spectrométrie de masse (SM): appareil JEOL D300; 70eV; impact électronique.
   Les valeurs sont exprimées en unités de masse atomique (m/z) et les intensités en % du pic de base sont indiquées entre parenthèses.
- Spectrographie infrarouge (IR) ; pastille KBr.
   Les valeurs sont exprimées en cm⁻¹. Les intensités des bandes, entre parenthèses, sont notées f (faible), m (moyenne), F (forte) et TF (très forte).
- Pouvoir rotatoire.
- Analyse élémentaire quantitative.

### EXEMPLE 1

### a) Préparation de la 4,6-O-isopropylidène-2,3-di-O-triméthylsilyl-D-glucono-1,5-lactone (1)

Dans un ballon contenant 100 ml de diméthylformamide anhydre, 84 mg d'acide paratoluène-sulfonique et 5g (28 mmoles) de D-glucono-1,5-lactone, maintenu à 0°C, on ajoute goutte à goutte 5,4 ml (56 mmoles) de 2-méthoxypropène.

Après 24 heures à 0°C, on ajoute 11,3 ml (140mmoles) de pyridine et, goutte à goutte, 11 ml (84 mmoles) de chlorotriméthylsilane.

Après 2 heures sous agitation à 20-25°C, on verse le contenu du ballon dans de l'eau glacée, le pH devant rester basique. Après extraction de la phase aqueuse avec de l'éther (2X 50 ml), les phases organiques sont rassemblées, séchées (sulfate de sodium), filtrées et évaporées. Le résidu obtenu est soumis à une étape de chromatographie sur gel de silice (éther de pétrole/acétate d'éthyle 95/5; v/v).

On récupère 6,6 g de 4,6-O-isopropylidène-2,3-di-O-triméthylsilyl-D-glucono-1,5-lactone (composé 1) sous la forme d'un solide blanc, soit un rendement de 65 % calculé sur la base de la masse de D-glucono-1,5-lactone de départ.

Les caractéristiques du composé 1 sont les suivantes : 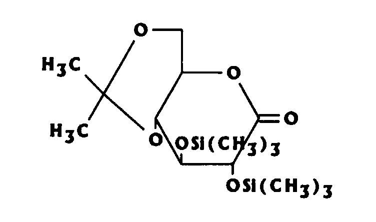
- point de fusion : 80 °C.
- RMN ¹H : 0,14 (s, 9H, Si(CH₃)₃) ; 0,20 (s, 9H, Si(CH₃)₃) ; 1,43 (s, 3H, CH₃) ; 1,50 (s, 3H, CH₃) ; 3,66-4,10 (m, 6H, H-2, H-3, H-4, H-5, H-6a, H-6e).
- RMN ¹³C : 0,1 (Si(CH₃)₃) ; 0,7 (Si(CH₃)₃); 18,8 (C(CH₃)) ; 28,7 (C(CH₃)) ; 61,5 (C-6) ; 68,7 (C-5) ; 72,6 (C-4) ; 75,8 (C-3 et C-2) ; 99,7 (Cq isopropylidène) ; 170,0 (C-1).
- SM : 362 (M⁺, 6) ; 342 (25) ; 304 (27) ; 289 (14) ; 215 (68) ; 157 (53) ; 73 (100).
- IR : 2961 (f) ; 1768 (F) ; 1253 (m) ; 1140 (m) ; 852 (F).

### b) Préparation du 1-C-perfluorobutyl-4,6-O-isopropylidène-2,3-di-O-triméthylsilyl-α-D-glucopyranose (3a)

Dans un ballon contenant une solution d'iodure de perfluorobutyle (6,6 mmoles dans 20 ml d'éther anhydre), placé à -45°C et à l'abri de la lumière, on ajoute goutte à goutte 2,2 ml (6,6 mmoles) de bromure d'éthylmagnésien.

Après 30 minutes, on introduit goutte à goutte 2 g (5,5 mmoles) du composé 1 obtenu à l'étape a) en solution dans 3 ml d'éther. On laisse la température remonter à -10°C. La réaction terminée, on ajoute une solution saturée de chlorure d'ammonium jusqu'à ce que le pH soit neutre.

La phase aqueuse est récupérée et extraite avec de l'éther (2 X 100 ml). Les phases organiques sont rassemblées, séchées (sulfate de magnésium) et évaporées. Le résidu solide obtenu est chromatographié sur gel de silice (éther de pétrole/acétate d'éthyle 95/5; v/v).

On récupère le 1-*C*-perfluorobutyl-4,6-*O*-isopropylidène-2,3-di-*O*-triméthylsilyl-a-D-glucopyranose (composé 3a) avec un rendement égal à 96 % calculé sur la base de la masse du composé 1 de départ.

Les caractéristiques du composé 3a sont les suivantes : 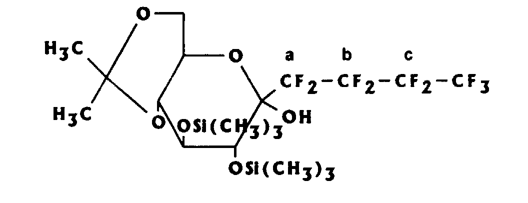
• point de fusion : 78°C.
• RMN ¹H : 0,14 (s, 9H, Si(CH₃)₃) ; 0,19 (s, 9H, Si(CH₃)₃) ; 1,41 (s, 3H, CH₃) ; 1,48 (s, 3H, CH₃) ; 3,44 (dd, 1H, J_{4,3}=J_{4,5}=9,1, H-4) ; 3,62 (dd, 1H, J_{3,4}=9,1, J_{3,2}=7,6, H-3) ; 3,72-3,79 (m, 2H, H-5, H-6e); 3,89 (dd, 1H, J_{6a,6e}=8,8, J_{6a,5}=1,2, H-6a) ; 4,04 (dd, 1H, J_{2,3}=7,6, J_{2,F}=1,4, H-2) ; 4,59 (sL, 1H, OH).
• RMN ¹³C : 0,5 (Si(CH₃)₃; 1,0 (Si(CH₃)₃; 18,8 (C(CH₃)) ; 28,9 (C(CH₃)) ; 61,5 (C-6); 64,0 (C-5) ; 72,3 (C-4) ; 73,5 (C-2); 75,9 (C-3); 97,8 (t, ²J_{C,F}=26,0, C-1) ; 99,5 (Cq isopropylidène).
• RMN ¹⁹F : -81,35 (t, 3F, J=10,2, CF₃) ; -120,61 (dm, 1F, J_{AB}=297,6, CFa) ; -120,83 (massif, 2F, CF₂b) ; -122,18 (dm, 1F, J_{AB}=297,6, CFa') ; -125,33 (dm, 1F, J_{AB}=305,2, CFc) ; -127,38 (dm, 1F, J_{AB}=305,2, CFc').
• SM : 582 (M⁺, 17) ; 434 (5) ; 345 (14) ; 204 (27) ; 144 (44) ; 103 (32) ; 73 (100).
• IR : 3537 (f) ; 2972 (f) ; 1379 (m) ; 1215 (F) ; 1140 (F) ; 852 (F).
• [α]²⁰_{D}=+9,7° (c=1,03 ; CHCl₃).
• Analyse : C₁₉H₃₁O₆Si₂F₉ (582,61)

| | | |
|---|---|---|
| Théorique | C : 39,17 | H : 5,32 |
| Calculé | C : 39,23 | H : 5,37 |

### c) Préparation du 1-C-perfluorobutyl-4,6-O-isopropylidène-α-D-gluco pyranose (4a)

Dans un ballon contenant 0,91 g (1,16 mmole) du composé 3a obtenu à l'étape précédente dissous dans 6 ml de méthanol, on ajoute 1,16 mmole de fluorure de tétrabutylammonium à 20-25°C.

Après deux heures sous agitation, on évapore le solvant. Le résidu obtenu est recristallisé dans le toluène. On récupère le 1-*C*-perfluorobutyl-4,6-*O*-isopropylidène-α-D-glucopyranose (composé 4a) avec un rendement de 96 % calculé sur la base de la masse du composé 3a de départ.

Les caractéristiques du composé 4a sont les suivantes : 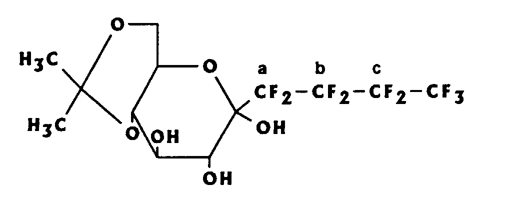
• point de fusion : 177°C
• RMN ¹H : 1,33 (s, 3H, CH₃) ; 1,48 (s, 3H, CH₃) ; 3,52-3,81 (massif, 5H, H-3, H-4, H-5, H-6ax, H-6eq) ; 3,91 (d, 1H, J=8,0, H-2) ; 4,56 (sL, 1H, OH) ; 4,98 (sL, 1H, OH) ; 6,14 (sL, 1H, OH anomérique).
• RMN ¹³C : 19,4 (C(CH₃)) ; 29,5 (C(CH₃)) ; 62,5 (C-6) ; 65,6 (C-5) ; 72,5 (C-4) ; 73,2 (C-2) ; 73,6 (C-3) ; 98,8 (t, ²J_{CF}=26,0, C-1) ; 100,2 (Cq isopropylidène).
• RMN ¹⁹F : -80,67 (t, 3F, J=9,0, CF₃) ; -120,16 (massif, 4F, CF₂a, CF₂b) ; -124,83 (dm, 1F, J_{AB}=290,0, CFc) ; -125,74 (dm, 1F, J_{AB}=290,0, CFc').
• SM : 438 (M^{+.}, trace) ; 423 (60) ; 315 (9) ; 299 (7) ; 109 (14) ; 73 (43) ; 59 (100).
• IR : 3412 (L) ; 2997 (f) ; 1390 (f) ; 1244 (F) ; 1140 (F) ; 713 (m).
• [α]¹⁹_{D}=+10,0° (c=0,45 ; CH₃COCH₃).
• Analyse : C₁₃H₁₅O₆F₉ (438,25)

| | | |
|---|---|---|
| Théorique | C : 35,62 | H : 3,45 |
| Calculé | C : 35,56 | H : 3,15 |

### d) Préparation du 1-C-perfluorobutyl-α-D-glucose (5a)

Dans un ballon surmonté d'un réfrigérant contenant 11,9 mmoles du composé 4a obtenu à l'étape précédente dissous dans 60 ml d'un mélange éthanol/eau (95/5 ; v/v), on ajoute 1,15 g de résine acide (Amberlyst 15 wet H⁺ ; ALDRICH).

Après 2 heures d'agitation au reflux de l'éthanol, on filtre le contenu du ballon. La phase organique est récupérée et évaporée. Le résidu obtenu, chromatographié sur gel de silice (éther de pétrole/acétate d'éthyle 20/80 ; v/v), fournit le 1-C-perfluorobutyl-D-glucose (composé 5a) avec un rendement de 95 % calculé sur la base de la masse du composé 4a de départ.

Le composé 5a est constitué d'un mélange d'une forme pyranique et d'une forme furanique dont les proportions varient en fonction du solvant de RMN utilisé :
- furane : pyrane = 30 : 70 (CD₃COCD₃)
- furane : pyrane = 80 : 20 (CD₃OD)

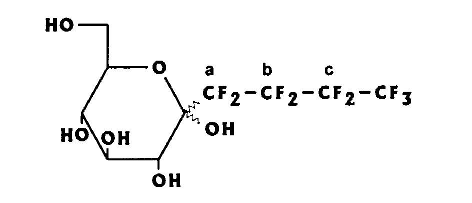

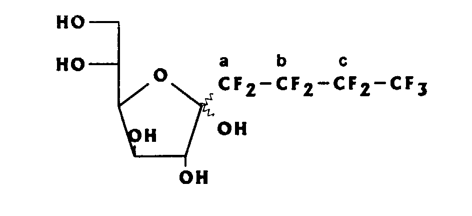

- point de fusion : 140-142°C.
- RMN ¹H : 3,68 (dd, 1H, J_{6,6'}=11,5, J_{6,5}=6,0, H-6) ; 3,85 (dd, 1H, J_{6',6}=11,5, J_{6',5}=3,2, H-6') ; 4;00 (m, 1H, H-5) ; 4,18 (dd, 1H, J_{4,5}=8,1, J_{4,3}=4,9, H-4) ; 4,37 (dd, 1H, J_{3,4}=4,9, J_{3,2}=2,8, H-3) ; 4,42 (d, 1H, J_{2,3}=2,8, H-2).
- RMN¹³C:

*Forme furanique :* 64,9 (C-6) ; 71,4 (C-5) ; 78,1 (C-3) ; 79,7 (C-2) ; 80,1 (C-4) ; 103,0 (t, ²J_{C,F}=29,0, C-11'; 110,8 (ts, J_{1,F}=270, J_{2,F}=39, Cc) ; 113,0 (tq, J_{1,F}=269, J_{2,F}=30, Cb) ; 115,5 (tt, J_{1,F}=261, J_{2,F}=30, Ca) ; 119,3 (qt, J_{1,F}=288, J_{2,F}=34, CF₃).

*Forme pyranique :* 62,7 (C-6) ; 71,0 (C-5) ; 72,8 (C-3) ; 75,3 (C-2) ; 76,2 (C-4) ; 98,6 (t, ²J_{C,F}=24,0, C-1).
• RMN ¹⁹F : -80,74 (t, 3F, ⁴J=9,0, CF₃) ; -119,83 (massif, 2F, CF₂a) ; - 120,50 (massif, 2F, CF₂b) ; -125,41 (massif, 2F, CF₂c).
• SM : 398 (M^{+.}, trace); 289 (17); 259 (16) ; 131 (34) ; 109 (32); 73 (100).
• IR : 3601 (m) ; 3474 (L) ; 3387 (L) ; 2961 (m) ; 1228 (TF) ; 1140 (TF) ; 1078 (m).
• [α]²²_{D}=+13,7° (c=0,46 ; CH₃OH)
• Analyse : C₁₀H₁₁O₆F₉ (398,18)

| | | |
|---|---|---|
| Théorique | C : 30,15 | H : 2,76 |
| Calculé | C : 30,59 | H : 2,59 |

### EXEMPLE 2

On procède dans les conditions de l'exemple 1 modifié en ce que l'on utilise l'iodure de perfluorohexyle. Dans cet exemple, le radical perfluoroalkyle est noté :

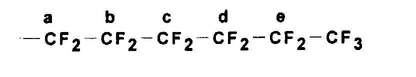

A l'issue de l'étape b, on obtient le 1-C-perfluorohexyl-4,6-O-isopropylidène-2,3-O-triméthylsilyl-α-D-glucopyranose (composé 3b) dont les caractéristiques sont les suivantes :
• point de fusion : 78°C.
• RMN ¹H : 0,16 (s, 9H, Si(CH₃)₃) ; 0,18 (s, 9H, Si(CH₃)₃) ; 1,41 (s, 3H, CH₃) ; 1,49 (s, 3H, CH₃) ; 3,44 (dd, 1H, J_{4,3}=J_{4,5}=9,1, H-4) ; 3,64 (dd, 1H, J_{3,4}=9,1, J_{3,2}=7,6, H-3) ; 3,69-3,84 (m, 2H, H-5, H-6e); 3,89 (dd, 1H, J_{6a,6e}=8,8, J_{6a,5}=4,2, H-6a) ; 4,04 (dd, 1H, J_{2,3}=7,6, J_{2,F}=1,6, H-2) ; 4,62 (sL, 1H, OH).
• RMN ¹³C : 0,5 (Si(CH₃)₃); 1,0 (Si(CH₃)₃); 18,8 (C(CH₃)) ; 28,9 (C(CH₃)) ; 61,5 (C-6); 64,0 (C-5) ; 72,4 (C-4) ; 73,5 (C-2); 75,9 (C-3); 97,8 (t, ²J_{C,F}=25,0, C-1) ; 99,5 (Cq isopropylidène).
• RMN ¹⁹F : -81,35 (t, 3F, J=9,0, CF₃) ; -119,79 (massif, 4F, CF₂a, CF₂b) ; -122,10 (massif, 2F, CF₂c) ; -123,20 (massif, 2F, CF₂d) ; -126,55 (massif, 2F, CF₂e).
• SM : 682 (M^{+.}, 48) ; 445 (26) ; 204 (42) ; 144 (100) ; 103 (60) ; 73 (78).
• IR : 3549 (f) ; 2947 (f) ; 1240 (F) ; 1153 (m) ; 852 (F).
• [α]²⁰_{D}=+8,0° (c=1,1 ; CHCl₃).
• Analyse : C₂₁H₃₁O₆Si₂F₁₃ (682,63)

| | | |
|---|---|---|
| Théorique | C : 36,95 | H : 4,54 |
| Calculé | C : 37,21 | H : 4,52 |

A l'issue de l'étape c, on obtient le 1-C-perfluorohexyl-4,6-*O*-isopropylidéne-α -D-glucopyranose (composé 4b) dont les caractéristiques sont les suivantes :
• point de fusion : 171°C.
• RMN ¹H : 1,33 (s, 3H, CH₃) ; 1,43 (s, 3H, CH₃) ; 3,53 (dd, 1H, J=9,5, J=1,5, H-4) ; 3,65-3,80 (m, 4H, H-3, H-5, H-6a, H-6e) ; 3,86 (dd, 1H, J=8,0, J=1,5, H-2); 4,43 (sL, 1H, OH) ; 4,87 (sL, 1H, OH) ; 5,97 (sL, 1H, OH anomérique).
• RMN ¹³C : 19,2 (C(CH₃)) ; 29,2 (C(CH₃)) ; 62,1 (C-6) ; 65,1 (C-5) ; 72,2 (C-4) ; 72,8 (C-2); 73,0 (C-3); 98,3 (t, ²J_{CF}=26,0, C-1) ; 99,9 (Cq isopropylidène) ; 106,8-124,7 (C₆F₁₃).
• RMN ¹⁹F : -80,99 (t, 3F, J=9,0, CF₃) ; -119,01 (massif, 4F, CF₂a, CF₂b) ; -121,04 (massif, 2F, CF₂c) ; -122,15 (massif, 2F, CF₂d) ; -125,63 (massif, 2F, CF₂e).
• SM : 538 (M^{+.}, 2) ; 523 (38) ; 137 (23) ; 95 (22) ; 69 (100).
• IR : 3425 (L) ; 2910 (f) ; 1390 (f) ; 1203 (F) ; 1153 (F).
• [α]¹⁹_{D}=+7,9° (c=0,39 ; CH₃COCH₃).
• Analyse : C₁₅H₁₅O₆F₁₃ (538,27)

| | | |
|---|---|---|
| Théorique | C : 33,45 | H : 2,78 |
| Calculé | C : 33,50 | H : 2,50 |

A l'issue de l'étape d, on obtient le 1-C-perfluorohexyl-D-glucose (composé 5b) avec un rendement de 95 % calculé sur la base de la masse du composé 4b de départ.

Les caractéristiques du composé 5b sont les suivantes :
- RMN ¹H : 3,68 (dd, 1H, J_{6,6'}=11,5, J_{6,5}=6,5, H-6) ; 3,84 (dd, 1H, J_{6',6}=11,5, J_{6',5}=3,2, H-6') ; 3,99 (m, 1H, H-5) ; 4,17 (dd, 1H, J_{4,5}=8,2, J_{4,3}=4,9, H-4) ; 4,36 (dd, 1H, J_{3,4}=4,9, J_{3,2}=2,8, H-3) ; 4,42 (d, 1H, J_{2,3}=2,8, H-2).
- RMN ¹³C :

*Forme furanique* : 64,9 (C-6) ; 71,4 (C-5) ; 78,2 (C-3) ; 79,8 (C-2) ; 80,2 (C-4) ; 103,1 (t, ²J_{C,F}=28,4, C-1).

*Forme pyranique* : 62,7 (C-6) ; 71,1 (C-5) ; 72,9 (C-3) ; 75,3 (C-2) ; 76,2 (C-4) ; 98,8 (t, ²J_{C,F}=25,0, C-1).
• RMN ¹⁹F : -80,74 (t, 3F, J=9,0, CF₃) ; -119,43 (massif, 4F, CF₂a, CF₂b) ; -121,06 (massif, 2F, CF₂c) ; -122,19 (massif, 2F, CF₂d) ; -125,04 (d, 1F, J_{AB}=310,0, CF₂e) ; -126,05 (d, 1F, J_{AB}=310,0, CF₂e').
• SM : 498 (M^{+.}, trace) ; 419 (10) ; 389 (22) ; 359 (17) ; 131 (28) ; 109 (41) ; 91 (20) ; 73 (100).
• IR : 3601 (f) ; 3387 (F) ; 2947 (f) ; 1203 (F) ; 1140 (F).
• [α]¹⁹_{D}=+10,5° (c=1,08 ; CH₃OH)
• Analyse : C₁₂H₁₁O₆F₁₃ (498,20)

| | | |
|---|---|---|
| Théorique | C : 28,91 | H : 2,20 |
| Calculé | C : 28,84 | H : 2,01 |

### EXEMPLE 3

On procède dans les conditions de l'exemple 1 modifié en ce que l'on utilise l'iodure de perfluorooctyle. Dans cet exemple, le radical perfluoroalkyle est noté :

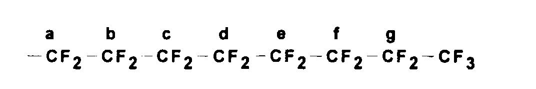

A l'issue de l'étape b, on obtient le 1-C-perfluorooctyl-4,6-O-isopropylidène-2,3-*O*-triméthylsilyl-α-D-glucopyranose (composé 3c) dont les caractéristiques sont les suivantes :
• point de fusion : 98-100°C.
• RMN ¹H : 0,12 (s, 9H, Si(CH₃)₃) ; 0,14 (s, 9H, Si(CH₃)₃) ; 1,38 (s, 3H, CH₃) ; 1,43 (s, 3H, CH₃) ; 3,41 (dd, 1H, J_{4,3}=9,1, J4,5=8,8, H-4) ; 3,61 (dd, 1H, J_{3,4}=9,1, J_{3,2}=7,6, H-3) ; 3,66-3,80 (m, 2H, H-5, H-6e) ; 3,85 (dd, 1H, J=8,8, J=4,2, H-6a) ; 4,01 (dd, 1H, J_{2,3}=7,6, J_{2,F}=1,5, H-2) ; 4,67 (sL, 1H, OH).
• RMN ¹³C: 0,4 (Si(CH₃)₃); 0,9 (Si(CH₃)₃); 18,8 (C(CH₃)) ; 28,8 (C(CH₃)) ; 61,5 (C-6); 64,0 (C-5) ; 72,3 (C-4) ; 73,5 (C-2); 75,9 (C-3); 97,8 (t, ²J_{C,F}=26,0, C-1) ; 99,5 (Cq isopropylidène).
• RMN ¹⁹F : -81,31 (t, 3F, J=9,0, CF₃) ; -120,31 (massif, 4F, CF₂a, CF₂b) ; -122,05 (massif, 6F, CF₂c, CF₂d, CF₂e) ; -123,11 (massif, 2F, CF₂f); - 126,55 (massif, 2F, CF₂g).
• SM : 782 (M^{+.}, 2) ; 767 (17) ; 601 (12) ; 545 (23) ; 204 (38) ; 159 (29) ; 131 (56) ; 103 (36) ; 73 (100).
• IR : 3387 (L) ; 2947 (m) ; 1203 (TF) ; 857 (F) ; 663 (f).
• [α]¹⁸_{D}=+10,6° (c=0,77; CH₃COCH₃).
• Analyse : C₂₃H₃₁O₆Si₂F₁₇ (782,65)

| | | |
|---|---|---|
| Théorique | C : 35,26 | H : 3,96 |
| Calculé | C : 34,93 | H : 3,88 |

A l'issue de l'étape c, on obtient le 1-*C*-perfluorooctyl-4,6-*O*-isopropylidène-α -D-glucopyranose (composé 4c) avec un rendement de 99 % calculé sur la base de la masse du composé 3c de départ.

Les caractéristiques du composé 4c sont les suivantes :
- point de fusion : 162°C.
- RMN ¹H : 1,34 (s, 3H, CH₃) ; 1,49 (s, 3H, CH₃) ; 3,57 (m, 1H, H-4) ; 3,75 (massif, 4H, H-3, H-5, H-6e, H-6a) ; 3,91 (d, 1H, J=8,4, H-2) ; 4,58 (sL, 1H, OH) ; 4,96 (sL, 1H, OH) ; 6,10 (sL, 1H, OH anomérique.)
- RMN ¹³C : 19,4 (C(CH₃)) ; 29,8 (C(CH₃)) ; 62,5 (C-6) ; 65,6 (C-5) ; 72,6 (C-4) ; 73,1 (C-2) ; 73,6 (C-3) ; 98,8 (t, ²J_{C,F}=25,0, C-1) ; 100,2 (Cq isopropylidène).
- RMN ¹⁹F : -80,74 (t, 3F, J=9,0, CF₃) ; -118,93 (massif, 4F, CF₂a, CF₂b) ; -121,09 (massif, 6F, CF₂c, CF₂d, CF₂e) ; -122,20 (massif, 2F, CF₂f); - 125,69 (massif, 2F, CF₂g).
- SM : 623 (68) ; 131 (24) ; 81 (31) ; 73 (39) ; 69 (64) ; 59 (100).
- IR : 3412 (L) ; 2997 (f) ; 1203 (F) ; 1153 (F) ; 1078 (m).
- [α]¹⁹_{D}=+6,5° (c=0,63; CH₃COCH₃).

A l'issue de l'étape d, on obtient le 1-C-perfluorooctyl-D-glucose (composé 5c) avec un rendement de 95 % calculé sur la base de la masse du composé 4c de départ.

Les caractéristiques du composé 5c sont les suivantes :
- point de fusion : 209°C.
- RMN ¹H : 3,68 (dd, 1H, J_{6,6'}=11,5, J_{6,5}=6,0, H-6) ; 3,84 (dd, 1H, J_{6',6}=11,5, J_{6',5}=3,2, H-6') ; 3,99 (m, 1H, H-5) ; 4,17 (dd, 1H, J_{4,5}=8,2, J_{4,3}=4,9, H-4) ; 4,36 (dd, 1H, J_{3,4}=4,9, J_{3,2}=2,8, H-3) ; 4,42 (d, 1H, J_{2,3}=2,8, H-2).
- RMN ¹³C

*Forme furanique* : 64,9 (C-6); 71,4 (C-5) ; 78,1 (C-3); 79,8 (C-2); 80,2 (C-4) ; 103,1 (t, ²J_{C,F}=28,8, C-1).

*Forme pyranique* : 62,7 (C-6) ; 71,1 (C-5) ; 72,9 (C-3) ; 75,3 (C-2) ; 76,2 (C-4) ; 98,8 (t, ²J_{C,F})=24,0, C-1).
• RMN ¹⁹F : -80,68 (t, 3F, J=9,0, CF₃) ; -119,42 (massif, 4F, CF₂a, CF₂b) ; -120,94 (massif, 6F, CF₂c, CF₂d, CF₂e) ; -122,16 (massif, 2F, CF₂f); - 125,16 (massif, 2F, CF₂g).
• SM : 562 (13) ; 519 (50) ; 139 (25) ; 109 (35) ; 73 (100).
• IR : 3601 (f) ; 3387 (F) ; 2961 (f) ; 1203 (F) ; 1140 (F); 1078 (m).
• [α]¹⁹_{D}=+7,9° (c=0,43 ; CH₃OH)
• Analyse : C₁₄H₁₁O₆F₁₇ (598,22)

| | | |
|---|---|---|
| Théorique | C : 28,09 | H : 1,83 |
| Calculé | C : 28,25 | H : 2,01 |

### EXEMPLE 4

### Préparation de la 4,6-O-isopropylidène-2,3-di-O-triméthylsilyl-D-glucono-1,5-lactone (1) et de la tétra-O-triméthylsilyl-D-glucono-1,5-lactone (2)

Dans un ballon contenant 65 ml de diméthylformamide anhydre, 60 mg d'acide paratoluène-sulfonique et 3,5 g (19,6 mmoles) de D-glucono-1,5-lactone, maintenu à 0°C, on ajoute goutte à goutte 3,8 ml (39,2 mmoles) de 2-méthoxypropéne.

Après 24 heures à 0°C, on ajoute 7,9 ml (98 mmoles) de pyridine et, goutte à goutte, 7,7 ml (5,9 mmoles) de chlorotriméthylsilane.

Après 2 heures sous agitation à 20-25°C, on verse le contenu du ballon dans de l'eau glacée, le pH devant rester basique. Après extraction de la phase aqueuse avec de l'éther (2 X 50 ml), les phases organiques sont rassemblées, séchées (sulfate de sodium), filtrées et évaporées. Le résidu obtenu contient la 4,6-O-isopropylidène-2,3-di-O-triméthylsilyl-D-glucono-1,5-lactone (composé 1, majoritaire), et la tétra-*O*-triméthylsilyl-D-glucono-1,5-lactone (composé 2).

### 1-C-perfluoroalkylation des composés 1 et 2

Dans un ballon contenant une solution d'iodure de perfluorobutyle (23,5 mmoles dans 80 ml d'éther anhydre), placé à -45°C et à l'abri de la lumière, on ajoute goutte à goutte 23,5 mmoles de bromure d'éthylmagnésien.

Après 30 minutes, on introduit goutte à goutte, 19,6 mmoles du résidu obtenu à l'étape précédente en solution dans 20 ml d'éther. On laisse la température remonter à -10°C et on ajoute une solution saturée de chlorure d'ammonium jusqu'à ce que le pH soit neutre.

La phase aqueuse est récupérée et extraite avec de l'éther (2 X 100 ml). Les phases organiques sont rassemblées, séchées (sulfate de magnésium) et évaporées. On obtient un résidu solide.

### Préparation du 1-C-perfluorobutyl-D-glucose

Dans un ballon surmonté d'un réfrigérant contenant 18,7 mmoles du résidu solide obtenu à l'étape précédente dissous dans 90 ml d'un mélange éthanol/eau (95:5; v/v), on ajoute 1,7 g de résine acide (Amberlyst 15 wet H⁺ ; ALDRlCH).

Après 2 heures d'agitation au reflux du mélange éthanol/eau, on filtre le contenu du ballon. La phase organique est récupérée et évaporée. Le résidu obtenu, chromatographié sur gel de silice (éther de pétrole/acétate d'éthyle 20/80; v/v), fournit 6,26 g de 1-C-perfluorobutyl-D-glucose (composé 5a) soit un rendement de 80 % calculé sur la base de la D-glucono-1,5-lactone de départ.

### EXEMPLE 5

### Préparation de la tétra-O-triméthylsilyl-D-glucono-1,5-lactone (2)

Dans un ballon contenant 5 g (28 mmoles) de D-glucono-1,5-lactone dissoute dans 47 ml de pyridine, on ajoute 23,6 ml (112 mmoles) d'hexaméthyldisilazane et 7 ml (56 mmoles) de chlorure de triméthylsilyle.

Après 2 heures d'agitation à 20-25°C, on ajoute 150 ml de pentane et on filtre le contenu du ballon sur de la célite afin d'éliminer les sels. Le filtrat est récupéré et évaporé. On obtient 13 g de tétra-O-triméthylsilyl-D-glucono-1,5-lactone (composé 2) soit un rendement de 100 % calculé sur la base de la masse de D-glucono-1,5-lactone de départ.

### Préparation du 1-C-perfluorohexyl-D-glucose (5b)

Dans un ballon contenant une solution d'iodure de perfluorohexyle (7,7 mmoles dans 35 ml d'éther anhydre), placé à -45°C et à l'abri de la lumière, on ajoute goutte à goutte 2,76 ml (7,7 mmoles) de bromure d'éthylmagnésien.

Après 30 minutes, on introduit goutte à goutte 3 g (6,43 mmoles) du composé 2 précité en solution dans 10 ml d'éther. On laisse la température remonter à-10°C et on ajoute une solution saturée de chlorure d'ammonium jusqu'à ce que le pH soit neutre.

La phase aqueuse est récupérée et extraite avec de l'éther (2 X 100 ml). Les phases organiques sont rassemblées, séchées (sulfate de magnésium) et filtrées.

Le filtrat est récupéré et évaporé. On obtient 4,81 g du dérivé 1-C-perfluorohexyl correspondant soit un rendement de 95 % calculé sur la base de la masse du composé 2 de départ.

Ce dérivé est chauffé au reflux du méthanol (20 ml) pendant 4 heures.

Le résidu obtenu après évaporation du méthanol est chromatographié sur gel de silice (éther de pétrole/acétate d'éthyle 20/80; v/v). On récupère 2,93 g de 1-C-perfluorohexyl-D-glucose (composé 5b) soit un rendement de 92 % calculé sur la base de la masse de la D-glucono-1,5-lactone engagée au départ.

## Revendications

1. Perfluoroalkyl-C- glycosides, caractérisés en ce qu'ils sont constitués d'un monosaccharide dont le carbone anomérique est lié directement à un radical perfluoroalkyle et à un groupe hydroxyle.

2. Perfluoroalkyl-*C*-glycosides selon la revendication 1, caractérisés en ce que le monosaccharide est un ose renfermant 4 à 7 atomes de carbone.

3. Perfluoroalkyl-C-glycosides selon la revendication 2, caractérisés en ce que l'ose est le glucose, le galactose, le gulose ou le mannose.

4. Perfluoroalkyl-C-glycosides selon l'une des revendications 1 à 3, caractérisés en ce que le radical perfluoroalkyle est choisi parmi les radicaux linéaires ou ramifiés renfermant de 2 à 12 atomes de carbone.

5. Procédé de préparation des perfluoroalkyl-C-glycosides selon les revendications 1 à 4 caractérisé en ce qu'il consiste à :
a - faire réagir une aldonolactone avec un agent de protection des groupes hydroxyles,
b - faire réagir le produit de l'étape a) avec un composé de formule R_{F}-M dans laquelle R_{F} représente un radical perfluoroalkyle linéaire ou ramifié renfermant de 2 à 12 atomes de carbone, et M représente Li ou MgX, X étant un halogène,
c - et déprotéger lesdits groupes hydroxyles.

6. Procédé selon la revendication 5, caractérisé en ce que l'aldonolactone renferme de 4 à 7 atomes de carbone.

7. Procédé selon la revendication 6, caractérisé en ce que l'aldonolactone est choisie parmi l'érythrono-1,4-lactone, la glucono-1,5-lactone, la glucoheptono-1,4-lactone, la galactono-1,4-lactone, la gulono-1,4-lactone et la mannono-1,4-lactone.

8. Procédé selon la revendication 5, caractérisé en ce que l'agent de protection est choisi parmi les composés susceptibles de former des groupements éthers et/ou cétaliques avec les groupes hydroxyles libres de l'aldonolactone.

9. Procédé selon la revendication 8, caractérisé en ce que l'agent est choisi parmi les halogénures d'alkyle, les halogénures de trialkylsilyle, les composés carbonylés et les éthers d'énol.

10. Procédé selon la revendication 5, caractérisé en ce que la déprotection est réalisée par hydrogénolyse ou par hydrolyse.

11. Procédé selon la revendication 10, caractérisé en ce que l'hydrolyse totale est effectuée au moyen d'un fluorure, d'un alcool chauffé au reflux ou d'une résine acide.

12. Procédé selon la revendication 10 caractérisé en ce que l'hydrolyse sélective des groupements éthers est effectuée au moyen d'un fluorure.

13. Utilisation des perfluoroalkyl-C-glycosides selon l'une des revendications 1 à 4 comme agents tensioactifs.

14. Utilisation des perfluoroalkyl-*C*-glycosides selon l'une des revendications 1 à 4 comme agents d'extinction des feux.
